# EUROPEAN PATENT APPLICATION

(11) **EP 4 501 399 A1**
(43) Date of publication of application: **05.02.2025**
(21) Application number: 23780532.0
(22) Date of filing: 28.03.2023
(51) Int. Cl.: A61N 5/10

(54) **NEUTRON CAPTURE THERAPY DEVICE AND COLLIMATOR**

(30) Priority: 29.03.2022 JP 2022053834
(71) Applicant: Sumitomo Heavy Industries, Ltd., Tokyo 141-6025 (JP)
(72) Inventor: BABA Tatsuo, Yokosuka-shi, Kanagawa 237-8555 (JP); NAKAMURA Taiki, Yokosuka-shi, Kanagawa 237-8555 (JP)
(74) Representative: Louis Pöhlau Lohrentz
(86) International application number: PCT/JP2023/012539
(87) International publication number: WO 2023/190523

(57) **Abstract**

A neutron capture therapy device includes an accelerator that generates a particle beam, a target that generates a neutron ray by reacting with the particle beam, a collimator that includes a hole portion penetrating in an irradiating direction of the neutron ray and forms an irradiation field of the neutron ray generated from the target, and a deceleration member that decelerates the neutron ray generated from the target, in which a part of the deceleration member is disposed in the hole portion of the collimator.

## Description

### Technical Field

The present disclosure relates to a neutron capture therapy device and a collimator.

### Background Art

For example, boron neutron capture therapy (BNCT) using a boron compound is known as neutron capture therapy that irradiates cancer cells with neutron rays to kill the cancer cells. In the boron neutron capture therapy, boron that is taken into cancer cells in advance is irradiated with neutron rays and the cancer cells are selectively destroyed using the scattering of heavy charged particles that is caused by the irradiation of boron with the neutron rays.

A neutron capture therapy device disclosed in PTL 1 decelerates neutron rays generated from a target with a deceleration member, forms an irradiation field with a collimator, and irradiates an irradiation target with the neutron rays. Accordingly, the neutron rays can be decelerated to an appropriate energy by the deceleration member.

### Citation List

### Patent Literature

[PTL 1] Japanese Unexamined Patent Publication No. 2020-146119

### Summary of Invention

### Technical Problem

Here, in order to reduce a burden on the irradiation target, it is desired to increase the intensity of the neutron rays and to shorten a treatment time. However, due to constraints such as the heat resistance of the accelerator and the target, there is a limit to the intensity of the particle beams with which the target can be irradiated, and ultimately, the intensity of the neutron rays generated from the target. For this reason, it is required to increase neutron flux with which the irradiation target is to be irradiated with a method other than increasing the intensity of the neutron rays generated from the target.

Therefore, an object of the present disclosure is to provide a neutron capture therapy device and a collimator that can shorten a treatment time by increasing neutron flux with which an irradiation target is to be irradiated.

### Solution to Problem

A neutron capture therapy device according to an aspect of the present disclosure includes an accelerator that generates a particle beam, a target that generates a neutron ray by reacting with the particle beam, a collimator that includes a hole portion penetrating in an irradiating direction of the neutron ray and forms an irradiation field of the neutron ray generated from the target, and a deceleration member that decelerates the neutron ray generated from the target, in which a part of the deceleration member is disposed in the hole portion of the collimator.

The neutron capture therapy device according to the aspect of the present disclosure includes a collimator that includes a hole portion penetrating in an irradiating direction of the neutron ray and forms an irradiation field of the neutron ray generated from the target. For this reason, the neutron ray passes through the hole portion of the collimator, and the irradiation target is irradiated with the neutron ray in the irradiation field corresponding to the shape of the collimator. Further, in a state where the neutron ray passes through the deceleration member corresponding to a predetermined distance from the target and is thus adjusted to an appropriate energy, the irradiation target is irradiated with the neutron ray. Here, a part of the deceleration member is disposed in the hole portion of the collimator. For this reason, the target can be disposed close to the irradiation target as much as the deceleration member is disposed in the hole portion of the collimator. As a distance between the target and the irradiation target is shorter, neutron flux can be increased. From the above, it is possible to shorten a treatment time by increasing neutron flux with which an irradiation target is to be irradiated.

At least a part of the collimator may be detachable. In this case, it is possible to easily adjust the size of the deceleration member that is disposed in the collimator by replacing the collimator.

The collimator may include a protrusion part protruding from a wall surface, and the deceleration member may be provided in the hole portion of the protrusion part. Since the deceleration member can be disposed even at a position closer to the irradiation target than the wall surface in this case, a distance between the target and the irradiation target can be shortened.

At least a part of the protrusion part may be detachable from the wall surface. Accordingly, a part of the collimator can be easily detached from the wall surface. That is, it is possible to easily adjust the size of the deceleration member that is disposed in the hole portion of the collimator.

The deceleration member may be disposed in the hole portion of the protrusion part over a range of a half or more of the protrusion part in the irradiating direction. Accordingly, since the deceleration member that is disposed in the hole portion of the protrusion part can be increased in size, a distance between the target and the irradiation target can be shortened by that much.

The wall surface may extend perpendicular to the irradiating direction. In this case, the protrusion part has a simple configuration in which the protrusion part protrudes in the irradiating direction.

A gamma ray blocking member may be disposed on a side facing an irradiation target in the hole portion of the collimator. The gamma ray blocking member is disposed on the downstream side of the deceleration member in the irradiating direction. Therefore, since the gamma ray blocking member is disposed on a side facing the irradiation target in the hole portion, a large space in which the deceleration member can be disposed can be ensured in the hole portion.

A collimator according to another aspect of the present disclosure is a collimator that forms an irradiation field of a neutron ray, and includes a hole portion that penetrates in an irradiating direction of the neutron ray, in which a deceleration member that decelerates the neutron is disposed in the hole portion.

According to the collimator of the present disclosure, it is possible to obtain the same actions and effects as those of the above-described neutron capture therapy device.

### Advantageous Effects of Invention

According to the present disclosure, it is possible to provide a neutron capture therapy device and a collimator that can shorten a treatment time by increasing neutron flux with which an irradiation target is to be irradiated.

### Brief Description of Drawings

Fig. 1 is a schematic diagram showing a neutron capture therapy device according to an embodiment of the present disclosure.
Fig. 2 is a schematic diagram of a neutron generating unit.
Fig. 3 is a schematic diagram of a neutron generating unit according to a comparative example.
Figs. 4A and 4B are cross-sectional views showing a collimator according to a modification example.
Figs. 5A and 5B are cross-sectional views showing a collimator according to a modification example.

### Description of Embodiments

A preferred embodiment of the present disclosure will be described in detail below with reference to the drawings.

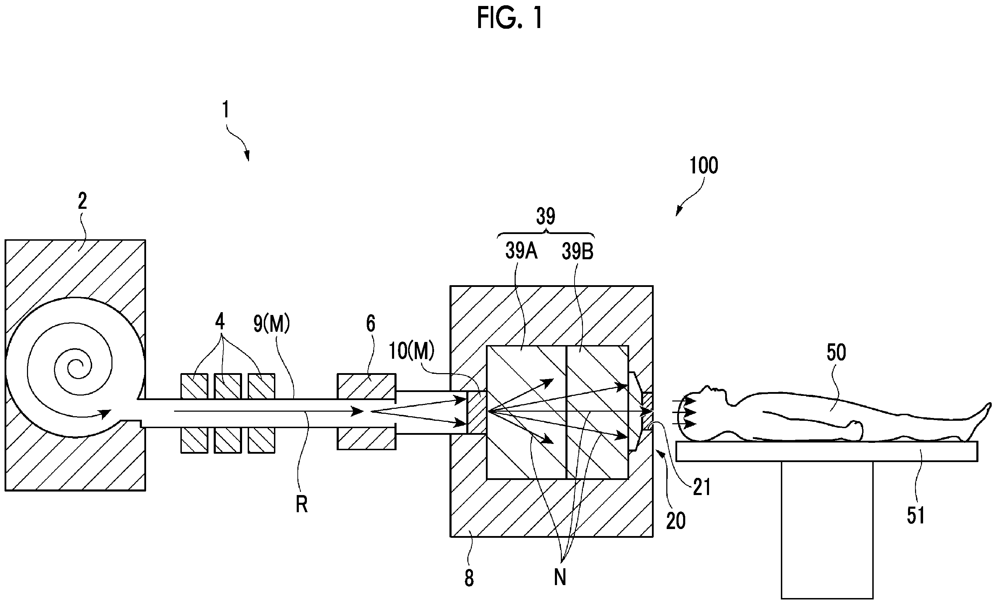

First, an outline of a neutron capture therapy device according to an embodiment of the present disclosure will be described with reference to Fig. 1. A neutron capture therapy device 1 shown in Fig. 1 is an apparatus that is used to treat cancer using boron neutron capture therapy (BNCT). In the neutron capture therapy device 1, for example, a tumor of a patient 50 who lies on a treatment table 51 to which boron (¹⁰B) is administered is irradiated with neutron rays N.

The neutron capture therapy device 1 includes an accelerator 2. The accelerator 2 accelerates charged particles such as negative ions to emit charged particle beams R. The accelerator 2 is formed of, for example, a cyclotron. In the present embodiment, the charged particle beam R is a proton beam that is generated in a case where charges are removed from negative ions. The accelerator 2 generates charged particle beams R having, for example, a beam radius of 40 mm and a power of 60 kW (= 30 MeV × 2 mA). The accelerator is not limited to the cyclotron, and may be a synchrotron, a synchrocyclotron, a linear accelerator, an electrostatic accelerator, or the like.

The charged particle beams R emitted from the accelerator 2 are sent to a neutron ray generating unit M. The neutron ray generating unit M includes a beam duct 9 (transport path) and a target 10. The charged particle beams R emitted from the accelerator 2 pass through the beam duct 9 and travel toward the target 10 disposed at an end portion of the beam duct 9. A plurality of quadrupole electromagnets 4 and a scanning electromagnet 6 are provided along the beam duct 9. The plurality of quadrupole electromagnets 4 are used, for example, to adjust the beam diameter of the charged particle beam R using an electromagnet.

The scanning electromagnet 6 is to scan the charged particle beams R and to control the irradiation of the target 10 with the charged particle beams R. The scanning electromagnet 6 controls a position at which the target 10 is irradiated with the charged particle beams R.

The neutron capture therapy device 1 includes a neutron generating unit 100 that irradiates the target 10 with the charged particle beams R to generate neutron rays N and emits the neutron rays N toward the patient 50. The neutron generating unit 100 includes the target 10, a shield member 8, a deceleration member 39, and a collimator 20.

The target 10 is to generate neutron rays N in a case where the target 10 is irradiated with the charged particle beams R. The target 10 is a solid member made of a material that generates neutron rays by being irradiated with charged particle beams. Specifically, the target 10 is made of, for example, beryllium (Be), lithium (Li), tantalum (Ta), or tungsten (W), and has, for example, the shape of a disk-shaped solid having a diameter of 160 mm. The target 10 is not limited to a disk shape and may have other shapes.

The deceleration member 39 is to decelerate the neutron rays N (is to reduce the energy of the neutron rays N) generated by the target 10. The deceleration member 39 may have a laminated structure including a layer 39A that mainly decelerates fast neutrons included in the neutron rays N and a layer 39B that mainly decelerates epithermal neutrons included in the neutron rays N. A substance having a large total cross-sectional area for neutrons having an electron-volt of 40 keV or more is employed as the material of the deceleration member 39. Specifically, examples of the material of the deceleration member 39 include CaF₂, MgF₂, AlF₃, D₂O, polyethylene, and the like.

The shield member 8 is to block the generated neutron rays N and gamma rays and the like generated with the generation of the neutron rays N so that the generated neutron rays N and the gamma rays and the like are not released to the outside. The shield member 8 is provided to surround the deceleration member 39. An upper portion and a lower portion of the shield member 8 extend to an upstream side of the charged particle beam R more than the deceleration member 39. A substance having a large total cross-sectional area for neutrons having an electron-volt of 0.5 eV or less is employed as a material of a neutron blocking member of the shield member 8. Examples of the material of the neutron blocking member include Li, boron, cadmium, polyethylene, and the like. A substance having a large total cross-sectional area for gamma rays is employed as a material of a gamma ray blocking member of the shield member 8. Examples of the material of the gamma ray blocking member include lead, iron, and the like.

The collimator 20 is to shape an irradiation field of the neutron rays N and includes an opening 20a through which the neutron rays N pass. The collimator 20 is, for example, a block-shaped member including a hole portion 21 at a center thereof.

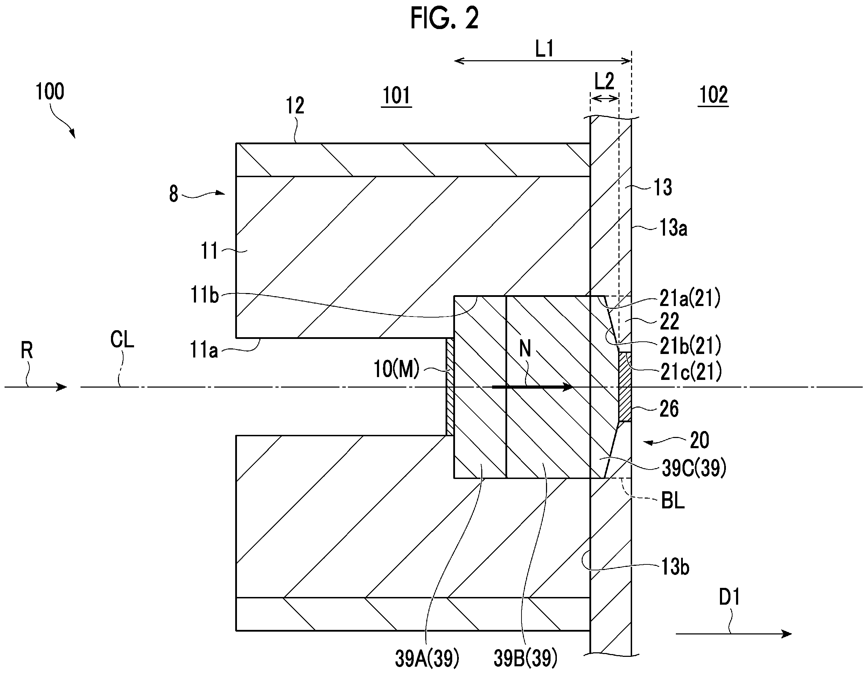

Next, a configuration of the neutron generating unit 100 will be described with reference to Fig. 2. In the following description, an irradiation axis of the charged particle beam R may be referred to as a reference line CL. The reference line CL corresponds to a center position in the irradiation with the neutron rays N. In a case where a direction is referred to as an "irradiating direction D1" in the following description, the direction refers to an irradiating direction of the charged particle beams R and the neutron rays N. As shown in Fig. 2, the shield member 8 includes a main body 11, an outer layer 12, and a wall part 13.

The main body 11 is disposed to surround the reference line CL. The main body 11 includes hole portions 11a and 11b extending along the reference line CL. The hole portion 11a is disposed on an upstream side in the irradiating direction D1, and the hole portion 11b is disposed on a downstream side. The hole portion 11a is an internal space that guides the charged particle beams R traveling toward the target 10. The hole portion 11b is an internal space in which the deceleration member 39 is disposed. The outer layer 12 is disposed on the outer periphery of the main body 11 in a direction orthogonal to the reference line CL.

The wall part 13 is disposed at an end portion of the main body 11 on the downstream side in the irradiating direction D1. The wall part 13 extends perpendicular to the reference line CL. The wall part 13 functions as a partition wall that separates an accelerator room 101 (see Fig. 1) in which the accelerator 2 is disposed and a treatment room 102 in which the treatment table 51 (see Fig. 1) is disposed. A downstream wall surface 13a of the wall part 13 in the irradiating direction D1 extends perpendicular to the irradiating direction D1 in the treatment room 102. An upstream wall surface 13b of the wall part 13 in the irradiating direction D1 extends perpendicular to the irradiating direction D1 in the accelerator room 101. The wall part 13 is connected to a floor at a lower end thereof and is connected to a ceiling at an upper end thereof. The floor and the ceiling also function as a shield member that blocks radiation.

The collimator 20 is formed in the wall part 13 at a position of the reference line CL. The hole portion 21 penetrating in the irradiating direction D1 is formed in the wall part 13, so that the collimator 20 is formed. The hole portion 21 includes inner peripheral surfaces 21a, 21b, and 21c in order from the upstream side to the downstream side in the irradiating direction D1. The inner peripheral surface 21a is a cylindrical surface having the same inner diameter as the inner diameter of the hole portion 11b of the main body 11. The inner peripheral surface 21b is a surface having an inclined cross-sectional shape such that an inner diameter thereof is reduced toward the downstream side in the irradiating direction D1. The inner peripheral surface 21c is a cylindrical surface having an inner diameter smaller than that of the inner peripheral surface 21a.

With such a configuration, the collimator 20 is adapted to include a narrowing portion 22 that protrudes from the inner peripheral surface 21a to an inner peripheral side. The narrowing portion 22 narrows the range of the irradiation field of the neutron rays N. The narrowing portion 22 may be formed as a member separate from the wall part 13 at a position of a boundary line BL. In this case, the narrowing portion 22 as a separate member is fitted to the inner peripheral surface 21a of the wall part 13. The narrowing portion 22 may be detachable from the wall part 13, or may be fixed not to be detachable. Alternatively, the narrowing portion 22 may be formed integrally with other portions of the wall part 13. In this case, the material of the wall part 13 is continuous without a cut at the position of the boundary line BL.

The layers 39A and 39B of the deceleration member 39 are provided in the hole portion 11b of the main body 11. The target 10 is disposed in the hole portion 11a of the main body 11.

The deceleration member 39 includes a layer 39C that is disposed in the hole portion 21 of the collimator 20, in addition to the layers 39A and 39B described above. The layer 39C is provided in the collimator 20 at a position corresponding to the inner peripheral surface 21a and the inner peripheral surface 21b. An outer peripheral surface of the layer 39C is provided to be in contact with the inner peripheral surfaces 21a and 21b of the hole portion 21. With such a configuration, a part of the deceleration member 39 is disposed in the hole portion 21 of the collimator 20.

The gamma ray blocking member 26 is disposed on a side facing the patient 50 in the hole portion 21 of the collimator 20. Specifically, the gamma ray blocking member 26 for blocking gamma rays is provided in the hole portion 21 at a position corresponding to the inner peripheral surface 21c. In a case where the narrowing portion 22 is detachable from the wall part 13, a portion of the layer 39C corresponding to the inner peripheral surface 21b and the gamma ray blocking member 26 may be detachable together with the narrowing portion 22.

Next, the actions and effects of the neutron capture therapy device 1 and the collimator 20 according to the present embodiment will be described.

The neutron capture therapy device 1 according to the present embodiment includes the collimator 20 that includes the hole portion 21 penetrating in the irradiating direction D1 of neutron rays N and forms the irradiation field of the neutron rays N generated from the target 10. For this reason, the neutron rays N pass through the hole portion 21 of the collimator 20 and the patient 50 is irradiated with the neutron rays N in the irradiation field corresponding to the shape of the collimator 20. Further, in a state where the neutron rays N pass through the deceleration member 39 corresponding to a predetermined distance from the target 10 and are thus adjusted to an appropriate energy, the patient 50 is irradiated with the neutron rays N.

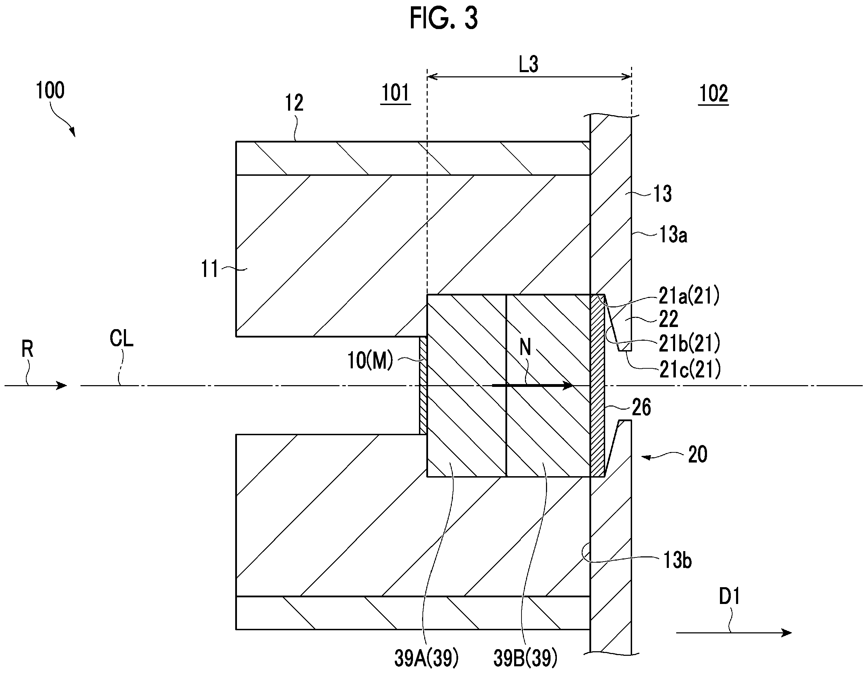

Here, a neutron capture therapy device according to a comparative example will be described with reference to Fig. 3. As shown in Fig. 3, the neutron capture therapy device according to the comparative example does not include the deceleration member 39 in the hole portion 21 of the collimator 20. The gamma ray blocking member is disposed on the most upstream portion of the inner peripheral surface 21a of the collimator 20 in the irradiating direction D1. A dimension between the target 10 and the wall surface 13a in this case in the irradiating direction D1 is L3.

On the other hand, in the neutron capture therapy device 1 according to the present embodiment, a part of the deceleration member 39 is disposed in the hole portion 21 of the collimator 20. For this reason, the target 10 can be disposed close to the patient 50 as much as the deceleration member 39 is disposed in the hole portion 21 of the collimator 20. For example, in Fig. 2, the layer 39C of the deceleration member 39 disposed in the hole portion 21 of the collimator 20 has a dimension L2. For this reason, a dimension L1 between the target 10 and the wall surface 13a in the irradiating direction D1 can be made shorter than the dimension L3 of the comparative example by the dimension L2. That is, in the present embodiment, the target 10 can be disposed closer to the patient 50 by the dimension L2 than in the comparative example. As a distance between the target 10 and the patient 50 is shorter, neutron flux can be increased. For example, as a result of performing a calculation on a model according to the example shown in Fig. 2 and a model according to the comparative example shown in Fig. 3, it was confirmed that neutron flux can be increased by about 10% in the model according to the example in a state where conditions other than the deceleration member 39 are the same as those in the model according to the comparative example. From the above, it is possible to shorten a treatment time by increasing the neutron flux with which the patient 50 is to be irradiated. Further, since a distance between the target 10 and the patient 50 can be shortened, the neutron capture therapy device 1 can be reduced in size.

At least a part of the collimator 20 may be detachable. In this case, it is possible to easily adjust the size of the deceleration member 39 that is disposed in the collimator 20 by replacing the collimator 20.

The gamma ray blocking member 26 may be disposed on a side facing the patient 50 in the hole portion 21 of the collimator 20. The gamma ray blocking member 26 is disposed on the downstream side of the deceleration member 39 in the irradiating direction D1. Therefore, since the gamma ray blocking member 26 is disposed on a side facing the patient 50 in the hole portion 21, a large space in which the deceleration member 39 can be disposed can be ensured in the hole portion 21.

The collimator 20 according to the present embodiment is a collimator 20 that forms the irradiation field of the neutron rays N and includes the hole portion 21 that penetrates in the irradiating direction D1 of the neutron rays N, and the deceleration member 39 that decelerates the neutron rays N is disposed in the hole portion 21.

According to the collimator 20 of the present disclosure, it is possible to obtain the same actions and effects as those of the above-described neutron capture therapy device 1.

The present disclosure is not limited to the above-described embodiment.

For example, the configuration of the neutron capture therapy device 1 described above is merely an example and can be appropriately changed.

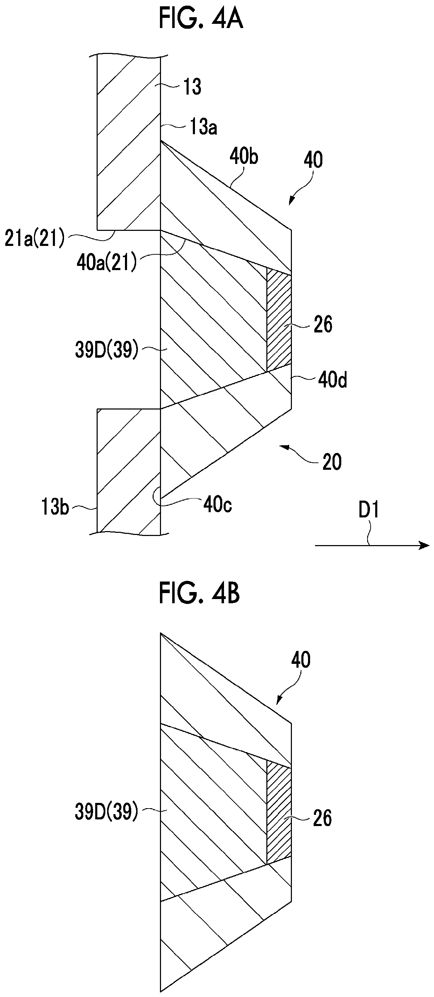

The structure of the collimator 20 is not limited to the above-described embodiment. For example, a collimator 20 shown in Figs. 4A and 4B may be employed. As shown in Fig. 4A, the collimator 20 includes an inner peripheral surface 21a of the wall part 13 and a protrusion part 40. The protrusion part 40 protrudes from the wall surface 13a of the wall part 13. The protrusion part 40 has a tubular structure having a truncated conical shape. The protrusion part 40 includes an upstream end portion 40c in the irradiating direction D1 and a downstream end portion 40d. The upstream end portion 40c is attached to the wall surface 13a in a state where the upstream end portion 40c is in contact with the wall surface 13a of the wall part 13. A diameter of an inner peripheral surface 40a is the same as the diameter of the inner peripheral surface 21a of the wall part 13. The inner peripheral surface 40a and an outer peripheral surface 40b are reduced in diameter toward the end portion 40d from the end portion 40c.

The gamma ray blocking member 26 is disposed on a side facing the patient 50 in the hole portion 21 of the protrusion part 40. The gamma ray blocking member 26 is disposed at a position of the downstream end portion 40d of the protrusion part 40. The layer 39D of the deceleration member 39 is provided in the hole portion 21 of the protrusion part 40. The layer 39D of the deceleration member 39 extends from the position of the upstream end portion 40c of the protrusion part 40 to the position of the gamma ray blocking member 26. The deceleration member 39 is disposed in the hole portion 21 of the protrusion part 40 over a range of a half or more of the protrusion part 40 in the irradiating direction D1.

The protrusion part 40 is detachable from the wall surface 13a. The downstream end portion 40c of the protrusion part 40 in the irradiating direction D1 can be detached from the wall surface 13a of the wall part 13. In this case, as shown in Fig. 4B, the layer 39D of the deceleration member 39 and the gamma ray blocking member 26 in the hole portion 21 of the protrusion part 40 can also be detached from the wall part 13 together with the protrusion part 40.

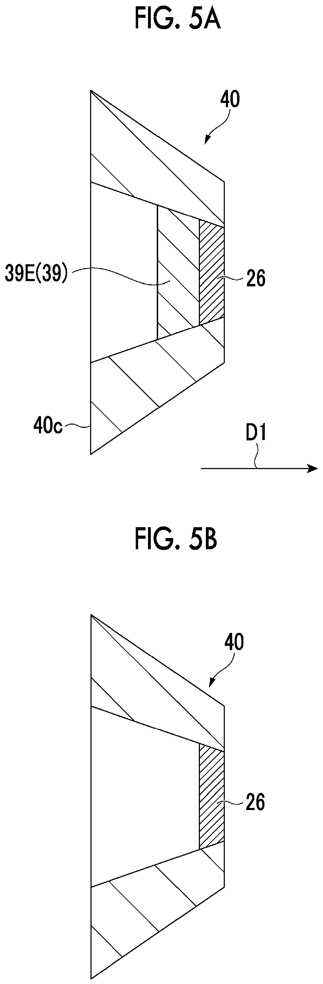

The protrusion part 40 attached to the wall part 13 is replaceable in accordance with the amount of the deceleration member 39 to be required. For example, the protrusion part 40 shown in Figs. 4A and 4B may be changed to a protrusion part 40 shown in Fig. 5A or a protrusion part 40 shown in Fig. 5B. The protrusion part 40 shown in Fig. 5A includes a layer 39E thinner than the layer 39D of the deceleration member 39 of the protrusion part 40 shown in Fig. 4B. The layer 39E is disposed at a position separated to a position spaced downstream from the end portion 40c of the protrusion part 40 in the irradiating direction D1. In this way, the deceleration member 39 may be disposed in the hole portion 21 of the protrusion part 40 over a range of a third or more of the protrusion part 40 in the irradiating direction D1. The protrusion part 40 shown in Fig. 5B does not include the deceleration member 39.

As described above, the collimator 20 may include the protrusion part 40 protruding from the wall surface 13a and the deceleration member 39 may be provided in the hole portion 21 of the protrusion part 40. Since the deceleration member 39 can be disposed even at a position closer to the patient 50 than the wall surface 13a in this case, a distance between the target 10 and the patient 50 can be shortened.

At least a part of the protrusion part 40 may be detachable from the wall surface 13a. Accordingly, a part of the collimator 20 can be easily detached from the wall surface 13a. That is, it is possible to easily adjust the size of the deceleration member 39 that is disposed in the hole portion 21 of the collimator 20.

The deceleration member 39 may be disposed in the hole portion 21 of the protrusion part 40 over a range of a half or more of the protrusion part 40 in the irradiating direction D1. Accordingly, since the deceleration member 39 that is disposed in the hole portion 21 of the protrusion part 40 can be increased in size, a distance between the target 10 and the patient 50 can be shortened by that much.

The wall surface 13a may extend perpendicular to the irradiating direction D1. In this case, the protrusion part 40 has a simple configuration in which the protrusion part 40 protrudes in the irradiating direction D1.

### Reference Signs List

- 1: neutron capture therapy device
- 2: accelerator
- 9: beam duct (transport path)
- 10: target
- 13a: wall surface
- 20: collimator
- 21: hole portion
- 26: gamma ray blocking member
- 39: deceleration member
- 40: protrusion part

## Claims

1. A neutron capture therapy device comprising:
an accelerator that generates a particle beam;
a target that generates a neutron ray by reacting with the particle beam;
a collimator that includes a hole portion penetrating in an irradiating direction of the neutron ray and forms an irradiation field of the neutron ray generated from the target; and
a deceleration member that decelerates the neutron ray generated from the target,
wherein a part of the deceleration member is disposed in the hole portion of the collimator.

2. The neutron capture therapy device according to claim 1,
wherein at least a part of the collimator is detachable.

3. The neutron capture therapy device according to claim 1,
wherein the collimator includes a protrusion part protruding from a wall surface, and
the deceleration member is provided in the hole portion of the protrusion part.

4. The neutron capture therapy device according to claim **3,**
wherein at least a part of the protrusion part is detachable from the wall surface.

5. The neutron capture therapy device according to claim **3,**
wherein the deceleration member is disposed in the hole portion of the protrusion part over a range of a half or more of the protrusion part in the irradiating direction.

6. The neutron capture therapy device according to claim 3,
wherein the wall surface extends perpendicular to the irradiating direction.

7. The neutron capture therapy device according to claim 1,
wherein a gamma ray blocking member is disposed on a side facing an irradiation target in the hole portion of the collimator.

8. A collimator that forms an irradiation field of a neutron ray, the collimator comprising:
a hole portion that penetrates in an irradiating direction of the neutron ray,
wherein a deceleration member that decelerates the neutron ray is disposed in the hole portion.
